(19) Europäisches Patentamt / European Patent Office / Office européen des brevets

(11) **EP 3 675 136 A1**

(12) **EUROPEAN PATENT APPLICATION**

(43) Date of publication:
01.07.2020 Bulletin 2020/27

(21) Application number: 19220106.9

(22) Date of filing: 30.12.2019

(51) Int Cl.:
G16H 40/63 (2018.01)    G16H 50/20 (2018.01)
G16H 50/70 (2018.01)    G16H 40/67 (2018.01)
G16H 20/40 (2018.01)    G16H 50/50 (2018.01)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 31.12.2018 US 201862786836 P
20.12.2019 US 201916723370

(71) Applicant: BIOSENSE WEBSTER (ISRAEL) LTD.
2066717 Yokneam (IL)

(72) Inventors:
• GOVARI, Assaf
2066717, Israel (IL)
• ALTMANN, Andres Claudio
2066717, Israel (IL)

(74) Representative: Small, Gary James
Carpmaels & Ransford LLP
One Southampton Row
London WC1B 5HA (GB)

(54) **DISEASE GUIDED INSERTION FOR IMPLANTS**

(57)    Methods, apparatus, and systems for medical procedures are disclosed herein and include receiving a medical condition indication, determining an optimal position for an implantable device based on the medical condition indication, guiding the implantable device from a non-optimal position to the optimal position based on determining the optimal position, and implanting the implantable device at the optimal position. The medical condition indication may be received from a remote computing system that may communicate with a local processor. The optimal position may include a location and an orientation and may be determined on the medical condition such that the optimal position may be best suited to sense biometric data based on the medical condition.

200

FIG. 2A

**Description**

CROSS REFERENCE TO RELATED APPLICATIONS

This application claims benefit of U.S. Patent Application Serial No. 62/786,836, filed December 31, 2018, which is incorporated by reference as if fully set forth.

FIELD OF INVENTION

The present application provides systems, apparatuses, and methods for improving medical procedures and recordings.

BACKGROUND

Medical conditions such as cardiac arrhythmia (e.g., atrial fibrillation (AF)) are often diagnosed and treated via intra-body procedures. For example, electrical pulmonary vein isolation (PVI) from the left atrial (LA) body is performed using ablation for treating AF. PVI, and many other minimally invasive catheterizations, cause damage to organ tissue to prevent electrical activity through the organ tissue.

Devices internal or external to the body may be used to sense activity such as intra-body organ activity. For example, intra-cardiac activity may be sensed either prior to or after a procedure. Such activity may be used to, for example, plan for a procedure or to sense the effects post a procedure. Accordingly, accurate sensing by such devices is needed to accurately plan for procedures and/or determine post procedure results.

SUMMARY

Methods, apparatus, and systems for medical procedures are disclosed herein and include receiving a medical condition indication, determining an optimal position for an implantable device based on the medical condition indication, guiding the implantable device from a non-optimal position to the optimal position based on determining the optimal position, and implanting the implantable device at the optimal position. The medical condition indication may be received from a remote computing system that may communicate with a local processor. The optimal position may include a location and an orientation and may be determined on the medical condition such that the optimal position may be best suited to sense biometric data based on the medical condition.

BRIEF DESCRIPTION OF THE DRAWINGS

A more detailed understanding can be had from the following description, given by way of example in conjunction with the accompanying drawings wherein:

FIG. 1 is a diagram of a system of the present invention;

FIG. 2A is a flowchart for generating implanting an implantable device at an optimal position;

FIG. 2B is a flowchart for determining an optimal position;

FIG. 3 is an illustration of a plurality of implantable device positions;

FIG. 4 is an illustration of a guide path to an optimal implantable device position;

FIGS. 5A and 5B are illustrations for providing directional information to guide an implantable device to an optimal position; and

FIG. 6 is a diagram of an example computer system including a remote computing system of the present invention.

DETAILED DESCRIPTION

Implantable devices such as implantable loop recorders (ILR) are devices which may be implanted into a patient, prior to or after a cardiac procedure. Such devices may be configured to record cardiac activity and, more specifically, to record abnormal cardiac events. However, traditional systems for implantation are not selective in terms of the position of the implant with relation to the heart, or of a section of the heart that may be of interest. Thus, such devices that are not selectively positioned may sense, record, and/or otherwise provide inefficient cardiac information that may be needed to be filtered or otherwise discarded by a healthcare professional. To clarify, traditional techniques for positioning an implantable device may include determining an educated estimate of the optimal position of the implantable device. However, such an educated estimate may not weigh multiple factors, a given patients anatomy, and or the specific source of the medical condition. Accordingly, an implantable device implanted in accordance with such an educated estimate may result in sub-optimal data and/or may result in data that needs to be further filtered to capture the desired amount and/or quality of data.

According to implementations of the disclosed subject matter, techniques and devices are provided for disease guided insertions of implants that allow for an implantable device to be optimally positioned based on one or more specific conditions. As disclosed herein, a medical condition indication may be received. The medical condition indication may be provided by a user (e.g., a medical professional) or may be automatically determined by a system (e.g., by a condition detection system. Alternatively, or in addition, the medical condition may be provided by a processor configured to receive and/or analyze data from one or more other components or systems.

The medical condition indication may be provided, for example, after a medical procedure such that the medical condition may either be known during the procedure or may be identified during the procedure. The techniques disclosed herein may be applied to determine the results of the medical procedure by using an implantable device. Alternatively, the medical condition indication may be provided, for example, prior to a medical procedure based on known or estimated conditions. The techniques disclosed herein may be applied to obtain information that informs a medical procedure.

The medical condition indication may be applied to determine an optimal position for the implantable device. The implantable device may be configured to sense biometric data (e.g., electrical activity) or other attributes of an organ, such as a heart. The medical condition indication may be applied to determine the optimal position such that the optimal position corresponds to a position that will provide the best sensed data by the implantable device, that is related to the respective medical condition.

Upon determination of the optimal position for the implantable device, the implantable device may be guided to the optimal position. The implantable device may be inserted into a human body via a natural orifice or via a cut or other laparoscopic incision. The implantable device may be considered to be in a non-optimal position until it is in an optimal position. The implantable device may be guided to the optimal position based on sensory indications (e.g., visual or sound based guidance), force feedback or haptic guidance, directional indications, or the like.

Upon reaching the optimal position, the implantable device may be implanted at the optimal position. The implantable device may be implanted at the optimal position in any applicable manner such as by an adhesive attachment, tissue insertion, mechanical attachment, or the like. The implantable device implanted at the optimal position may provide biometric data which may be optimized for the corresponding medical condition on which the optimal position is based on.

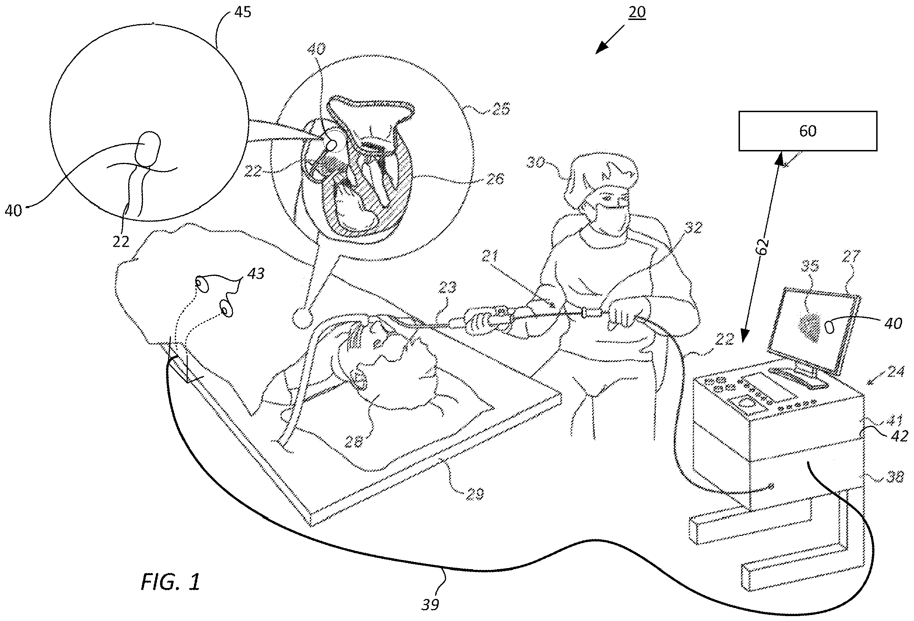

FIG. 1 is a diagram of an exemplary system 20 in which one or more exemplary features of the present invention can be implemented. System 20 may include components, such as an implantable device 40, that are configured to be implanted in areas of an intra-body organ. The implantable device 40 may also be configured to obtain biometric data and may include components, such as one or more electrodes, configured to sense, record, or otherwise capture biometric data. Although implantable device 40 is shown to be a single component, it will be understood that an implantable device of any shape that includes one or more components may be used to implement the implementations disclosed herein. System 20 includes a probe 21, having shafts that may be navigated by a medical professional 30 into a body part, such as heart 26, of a patient 28 lying on a bed 29. According to embodiments, multiple probes may be provided. For purposes of conciseness, a single probe 21 is described herein, but it will be understood that probe 21 may represent multiple probes. As shown in FIG. 1, medical professional 30 may insert shaft 22 through a sheath 23, while manipulating the distal end of the shaft 22 using a manipulator 32 near the proximal end of the implantable device 40 and/or deflection from the sheath 23. As shown in an inset 25, implantable device 40 may be fitted at the distal end of shaft 22. Implantable device 40 may be inserted through sheath 23 in a collapsed state and may be then expanded within heart 26. Upon implanting the implantable device 40, the shaft 22 may detach from the implantable device 40 and shaft 22 and any component of probe 21 may be removed from the patient 28 such that the implantable device 40 remains within the patient's body. It will be understood that the implantable device 40 may be wirelessly controlled and inserted into a patient's body without the use of a shaft 22.

According to embodiments, implantable device 40 may be configured to sense biometric data. Inset 45 shows implantable device 40 in an enlarged view, inside a cardiac chamber of heart 26. According to embodiments disclosed herein, biometric data may include one or more of LATs, electrical activity, topology, bipolar mapping, dominant frequency, impedance, or the like. The local activation time may be a point in time of a threshold activity corresponding to a local activation, calculated based on a normalized initial starting point. Electrical activity may be any applicable electrical signals that may be measured based on one or more thresholds and may be sensed and/or augmented based on signal to noise ratios and/or other filters. A topology may correspond to the physical structure of a body part or a portion of a body part and may correspond to changes in the physical structure relative to different parts of the body part or relative to different body parts. A dominant frequency may be a frequency or a range of frequency that is prevalent at a portion of a body part and may be different in different portions of the same body part. For example, the dominant frequency of a pulmonary vein of a heart may be different than the dominant frequency of the right atrium of the same heart. Impedance may be the resistance measurement at a given area of a body part.

As shown in FIG. 1, the probe 21 and implantable device 40 may be connected to a console 24 via a wire or wirelessly. Console 24 may include a processor 41, such as a general-purpose computer, with suitable front end and interface circuits 38 for transmitting and receiving signals to and from the implantable device 40, as well as for controlling the other components of system 20. In some embodiments, processor 41 may be further configured to receive biometric data, such as electrical activity, and determine if a given tissue area conducts electricity. According to an embodiment, the processor 41 may be external to the console 24 and may be located, for example, in the implantable device 40, in an external device, in a mobile device, in a cloud-based device, or may be a standalone processor.

As noted above, processor 41 may include a general-purpose computer, which may be programmed in software to carry out the functions described herein. The software may be downloaded to the general-purpose computer in electronic form, over a network, for example, or it may, alternatively or additionally, be provided and/or stored on non-transitory tangible media, such as magnetic, optical, or electronic memory. The example configuration shown in FIG. 1 may be modified to implement the embodiments disclosed herein. The disclosed embodiments may similarly be applied using other system components and settings. Additionally, system 20 may include additional components, such as elements for sensing electrical activity, wired or wireless connectors, processing and display devices, or the like.

According to an embodiment, a display 27 connected to a processor (e.g., processor 41) may be located at a remote location such as a separate hospital or in separate healthcare provider networks. Additionally, the system 20 may be part of a surgical system that is configured to obtain anatomical and electrical measurements of a patient's organ, such as a heart, and performing a cardiac ablation procedure. An example of such a surgical system is the Carto® system sold by Biosense Webster.

The system 20 may also, and optionally, obtain biometric data such as anatomical measurements of the patient's heart using ultrasound, computed tomography (CT), magnetic resonance imaging (MRI) or other medical imaging techniques known in the art. The system 20 may obtain electrical measurements using implantable devices such as catheters, electrocardiograms (EKGs) or other sensors that measure properties (e.g., electrical properties) of a body organ (e.g., a heart). The biometric data such as anatomical and electrical measurements may then be stored in a memory 42 of the mapping system 20, as shown in FIG. 1. The biometric data may be transmitted to the processor 41 from the memory 42. Alternatively, or in addition, the biometric data may be transmitted to a server 60, which may be local or remote, using a network 62. Similarly, ultrasound slices may be transmitted to a server 60, which may be local or remote, using a network 62. According to an embodiment, server 60 may be a remote system that may receive the biometric data and may send one or more signals to change a configuration or position of the implantable device 40. The one or more signals may be based on the biometric data received at the server 60.

Network 62 may be any network or system generally known in the art such as an intranet, a local area network (LAN), a wide area network (WAN), a metropolitan area network (MAN), a direct connection or series of connections, a cellular telephone network, or any other network or medium capable of facilitating communication between the mapping system 20 and the server 60 and/or a remote computing system. The network 62 may be wired, wireless or a combination thereof. Wired connections may be implemented using Ethernet, Universal Serial Bus (USB), RJ-11 or any other wired connection generally known in the art. Wireless connections may be implemented using Wi-Fi, WiMAX, and Bluetooth, infrared, cellular networks, satellite or any other wireless connection methodology generally known in the art. Additionally, several networks may work alone or in communication with each other to facilitate communication in the network 62.

In some instances, the server 62 may be implemented as a physical server. In other instances, server 62 may be implemented as a virtual server a public cloud computing provider (e.g., Amazon Web Services (AWS) ®).

Control console 24 may be connected, by a cable 39, to body surface electrodes 43, which may include adhesive skin patches that are affixed to the patient 28. The processor 41, in conjunction with a current tracking module (not shown), may determine position coordinates of the implantable device 40 inside the body part (e.g., heart 26) of a patient. The position coordinates may be based on impedances or electromagnetic fields measured between the body surface electrodes 43 and the electrode 48 or other electromagnetic components of the implantable device 40. Additionally, or alternatively, location pads may be located on the surface of bed 29 and may be separate from the bed 29.

Processor 41 may comprise real-time noise reduction circuitry typically configured as a field programmable gate array (FPGA), followed by an analog-to-digital (A/D) ECG (electrocardiograph) or EMG (electromyogram) signal conversion integrated circuit. The processor 41 may pass the signal from an A/D ECG or EMG circuit to another processor and/or can be programmed to perform one or more functions disclosed herein.

Control console 24 may also include an input/output (I/O) communications interface that enables the control console to transfer signals from, and/or transfer signals to electrode 47.

During a procedure, processor 41 may facilitate the presentation of a body part rendering 35 to medical professional 30 on a display 27, and store data representing the body part rendering 35 in a memory 42. The display 27 (or a remote display) may also be provided the implantable device 40's location. As further disclosed herein, the display 27 or other remote display may also display visual guidance to direct the implantable device 40 to an optimal position. Memory 42 may comprise any suitable volatile and/or non-volatile memory, such as random-access memory or a hard disk drive. In some embodiments, medical professional 30 may be able to manipulate a body part rendering 35 using one or more input devices such as a touch pad, a mouse, a keyboard, a gesture recognition apparatus, or the like. For example, an input device may be used to change the position of implantable device 40 such that rendering 35 is updated. In alternative embodiments, display 27 may include a touchscreen that can be configured to accept inputs from medical professional 30, in addition to presenting a body part rendering 35.

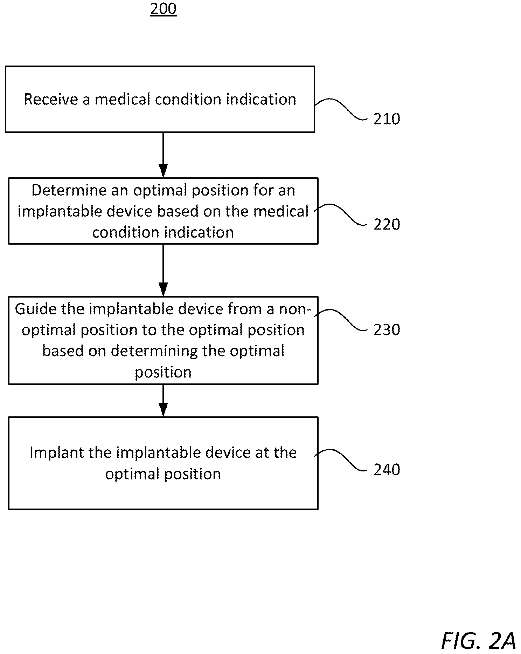

FIG. 2A shows a process 200 for implanting an implantable device (e.g., implantable device 40 of FIG. 1) at an optimal position. Notably, the process 200 of FIG. 2A may allow for an implantable device to be implanted at an optimal position that is optimized based on a given medical condition indication. The optimal position may allow for biometric data to be sensed by the implantable device, such that such biometric data is superior to sensed biometric data that may be obtained by the implantable device if it was not in the optimal position. To clarify, different medical conditions may require sensing different signals (e.g., different types of signals, signals that propagate in different directions, signals that are strongest at certain positions and not others, etc.) Accordingly, an implantable device that is implanted at an optimal position that is optimized based on a given medical condition may allow for improved data retrieval for that medical condition, as sensed by the implantable device.

At step 210 of the process 200 of FIG. 2A, a medical condition indication may be received. The medical condition indication may be received, for example, prior to or after a medical procedure.

A medical condition indication received post a medical procedure, may be received based on a known medical condition, a medical condition identified during the procedure, a location of a medical condition identified prior to, during, or post the procedure, or the like. As an example, an arrythmia at a given location of a heart chamber may be identified during a medical procedure. Accordingly, the medical condition indication may include or may be based on the type of arrythmia and/or the location of the arrythmia. A medical condition indication received prior to a medical procedure may be received based on a known or assumed medical condition. As an example, a patient's symptoms may indicate atrial fibrillation and, accordingly, the medical condition indication may indicate an atrial fibrillation.

The medical condition indication may be received by a user input, an automatic determination, or the like. For example, a medical professional may input a medical condition or other code that indicates the medical condition. Alternatively, a diagnostic system may determine that a patient exhibits a specific medical condition and may provide the medical condition indication based on the determination. For example, a diagnostic system or a physician may identify a rotor pattern at an area of the heart as sensed based on electrical activity recorded by electrodes of a catheter in communication with the diagnostic system. Based on the rotor pattern, the diagnostic system may determine that the location of the rotor pattern is a likely source of arrythmia and, thus, may provide a medical condition indication of arrythmia at that particular location.

The medical condition indication may be provided in any applicable format such as a numerical value (e.g., where a given numerical value corresponds to a given medical condition), an electronic signal, a code value, or a combination thereof. For example, a user may provide a medical condition indication of an arrhythmia within an area of a heart chamber and a processor (e.g., processor 41) may convert such information into a code that represents the arrhythmia and the location of the area of the heart.

At step 220 of the process 200 of FIG. 2A, an optimal position for an implantable device may be determined based on the medical condition indication. The optimal position may include one or more of a location, an orientation, an angle, an elevation, a depth, or the like. The optimal position based on the medical condition indication may be determined on one or more factors including the type of implantable device, noise at different positions, potential signal amplitudes, potential morphologies, and the like.

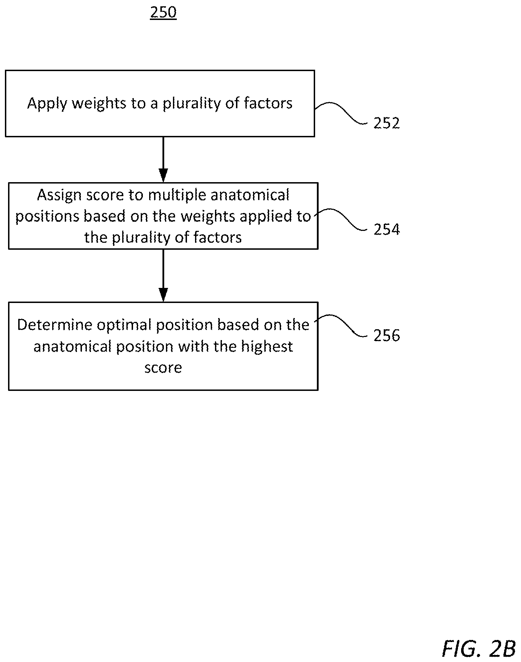

The optimal position for an implantable device may be determined based on a received or determined medical condition indication in view of the anatomy of a body organ, such as a heart. FIG. 2B shows a process 250 for determining an optimal position in accordance with the subject matter disclosed herein. As shown at step 252 of process 250, based on the type of medical condition indication, the system (e.g., via processor 41 of FIG. 1) may apply weights to a plurality of factors such that factors that are more important in view of the medical condition are weighted higher than factors that are less important in view of the medical condition.

As an example, electrical signals may be observed to identify an area of the heart causing atrial fibrillation (AFib) based on a rotor pattern exhibited at the area of the heart. The area of the heart may be ablated and an implantable device may be required to observe the ablated area of the heart to observe whether electrical activity persists at that area of the heart. Accordingly, a medical condition indication may indicate an AFib driven ablation at the area of the heart that exhibited the rotor pattern and, accordingly, a signal to noise ratio may be determined to be an important factor to determine if the ablation was successful (i.e., a signal exhibiting no electrical activity given a low signal to noise ratio may indicate a definitive lack of electrical activity). Accordingly, the signal to noise factor may be weighted more heavily than a signal amplitude factor.

At step 254 of process 250 of FIG. 2B, a score may be determined for a plurality of anatomical positions based on the weighted factors and sub-scores for each of the weighted factors. The score for each anatomical position may be based on, for example, the location and one or more orientations associated with each given anatomical position. Further, the score may be based on how each given anatomical position contributes to each factor, multiplied by the weight of each factor. As an example, Equation 1 shows the calculation of a score "S" for a given anatomical position "p" for "n" number of factors:

$$S_p = F_1 * SF_1 * WF_1 + \cdots + F_n * SF_n * WF_n, \quad (1)$$

In Equation 1 above, F refers to a factor (e.g., signal amplitude, modality, signal to noise ratio, etc.), SF refers to a sub-score for a given factor (e.g., signal amplitude at a given anatomical location), and WF refers to the weight for a given factor as determined based on the medical condition indication. It will be understood that a given anatomical location may result in multiple anatomical positions based on one or more orientations, depths, angles, or the like for the given anatomical location. As disclosed herein, a traditional technique using an educated estimate to determine placement of an implantable device may not consider at least such orientations, depths, angles, or the like as they may be medical condition indication specific, patient specific, patient anatomy specific, and/or may vary by small but analytically significant increments.

Continuing the previous example, a score may be applied to a plurality of anatomical positions of the patient's heart, based on the AFib medical condition indication as well as the highly weighted signal amplitude factor. A score may be determined for each anatomical position based on a summation or other operation of the sub-scores based on each weighted factor, including the highly weighted signal amplitude factor.

At step 256 of process 250 of FIG. 2B, an optimal position may be determined based on the anatomical position with the highest overall score. The optimal position may correspond to one or more of a location, an orientation, a depth, an angle, or the like. Continuing the previous example, the anatomical position that corresponds to the highest overall score given the AFib medical condition may be selected as the optimal position for the implantable device.

The optimal position may be determined to optimize the biometric data that is to be sensed and is most relevant to the corresponding medical condition. Accordingly, the optimal position for a given medical condition may vary from a different medical condition. Notably, the implantable device may have a goal operation such that, based on the medical condition, a medical professional or medical system may require several different types or qualities of biometric data from the implantable device. Accordingly, the optimal position based on a medical condition indication may be determined such that the implantable device can sense biometric data that is closest to the goal operation of the implantable device for that medical condition.

To clarify, as indicated at step 254 of the process 250 of FIG. 2B, and discussed herein with Equation 1, an overall score for each of a plurality of anatomical positions may be calculated based on weighted factors applied to sub-scores for each factor. A factor sub-score (e.g., a score for signal amplitude at a given anatomical position) may be determined for a given anatomical position based on the quality or quantity of that factor at the given location. As an example, for a given anatomical position, a sub-score may be determined for the signal amplitude factor based on a medical condition location (e.g., ablated area of the heart) as indicated by a medical condition indication. The system may determine that, based on an analysis of the patient's body anatomy as received based on an X-ray scan, an electrode based mapping, or a stored anatomy, that the signal amplitude sub-score for signals from the ablated area to the given anatomical position is .6. The system may determine that, based on an analysis of the patient's body anatomy, that the signal amplitude sub-score for signals from the ablated area to a different anatomical position is .8. In this simplified example, the different anatomical position may be more likely to be determined as the optimal position especially if signal amplitude factor is weighted heavily.

As another example of the subject matter disclosed herein, an arrhythmia based medical condition may be indicated and may originate at a first location of a heart. The processor 41 of FIG. 1 may receive the corresponding medical condition indication and may determine a plurality of anatomical positions based on the arrhythmia based medical condition and the first location. The processor 41 may access data, a look-up table, an algorithm, or the like which may be stored in memory 42 to determine the factors and corresponding weights for factors based on the arrhythmia based medical condition and the first location. The plurality of anatomical positions may be determined based on one or more factors such as the type of implantable device, noise at different positions, potential signal amplitudes, potential morphologies, and the like, or a combination thereof. Based on a plurality of the anatomical positions, the processor 41 may determine an optimal position, in accordance with process 250 of FIG. 2B, based on one or more other factors .

According to implementations of the disclosed subject matter, an optimal position may be identified based on one or more weights associated with one or more potential positions and/or one or more factors. The weights may be determined based on the medical condition indication. For example, a first medical condition may require analyzing a low amplitude signal and, thus may weight a higher signal to noise ratio greater than, for example, morphology of a given signal. According to this example, the optimal position may be one that will result in a greater signal to noise ratio.

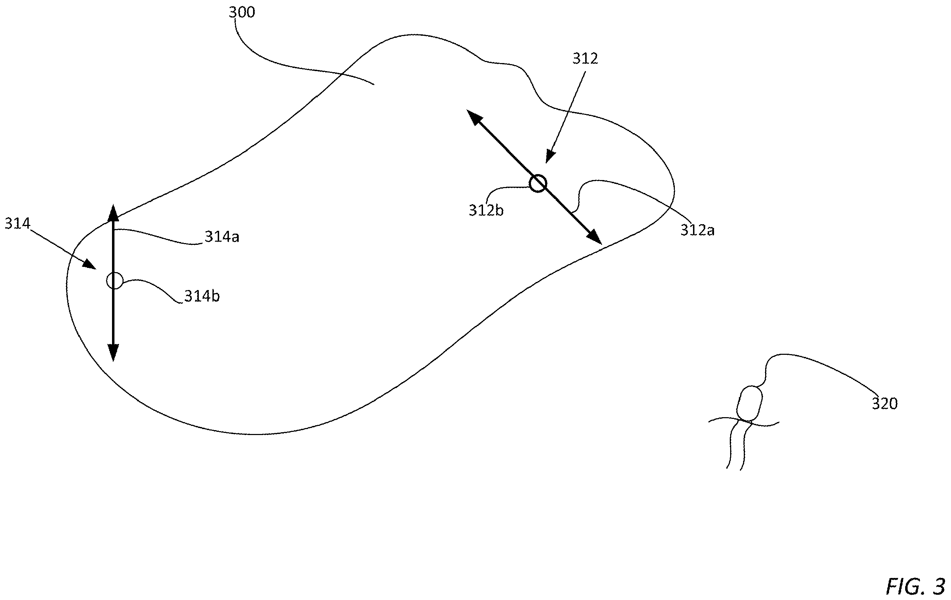

FIG. 3 shows an example of a plurality of positions 312 and 314 on a heart organ 300. Each of the plurality of positions 312 and 314 may have a location (e.g., 312a and 314a) and a plurality of orientations (e.g., 312b and 314b). It will be understood that a single location (e.g., 312a or 314a) may have a plurality of orientations associated with the location. It will also be understood that although a location and position are disclosed in this example, other attributes such as angle, elevation, depth, or the like may be utilized. A medical condition indication related to the heart organ 300 may be received. Based on the medical condition indication, a processor (e.g., processor 41 of FIG. 1) may determine that position 312 with location 312a and orientation 312b is the optimal position for an implantable device 320 based on the received medical condition indication. The position 312 with location 312a and orientation 312b may be determined to be the optimal position based on position 312 being better than one or more other positions (e.g., position 314) to sense the biometric data required for the given medical condition. For example, position 314 may provide electrical signals for the heart organ 300 that are not mixed with one or more other signals, reducing the probability of sensing errant signals.

At step 230 of the process 200 of FIG. 2A, the implantable device (e.g., implantable device 40 of FIG. 1) may be guided from a non-optimal position to an optimal position based on determining the optimal position at step 220. The implantable device may be guided based on providing a route to the optimal position, on providing directions to the optimal position, providing haptic feedback to the optimal position, or a combination thereof.

According to an example, an intra-body chamber such as a heart may be mapped using an electrode based mapping system. The electrode based mapping system may sense the boundaries of the chamber using tissue proximity indications (TPI) to detect the boundaries. A medical procedure may be performed within the chamber by referencing a rendering of the mapped chamber. Subsequent to the procedure, the medical condition being addressed by the procedure may be provided and an optimal location for an implantable device may be determined based on a medical condition indication. At step 230 of the process 200 of FIG. 2A, an implantable device may be rendered on the chamber rendering generated during the medical procedure and the same rendering may be used to guide the implantable device to the optimal position.

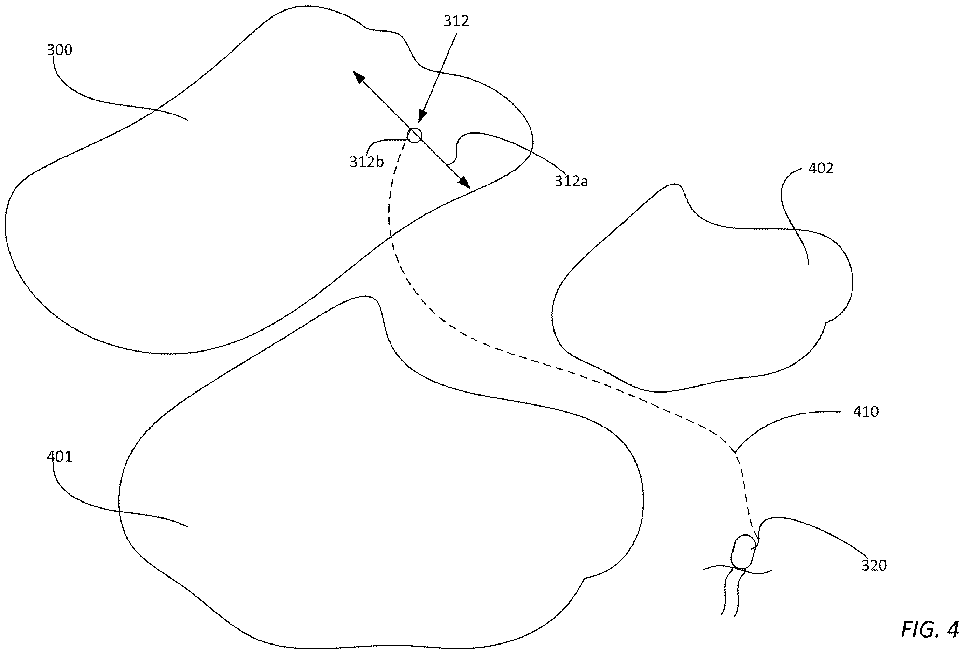

FIG. 4 shows the example of heart organ 300 provided in FIG. 3. For conciseness, the components of FIG. 3 that are also provided in FIG. 4 are not described again specifically in reference to FIG. 4. As shown, an optimal position 312 with location 312a and orientation 312b may be determined based on a given medical condition indication. A processor (e.g., processor 41 of FIG. 1) may provide a route 410 from a non-optimal position of the implantable device 320 to the determined optimal position 312 of the implantable device 320. A route to an optimal position may be any one of a shortest distance from a non-optimal position to an optimal position, a quickest time to get from a non-optimal position to an optimal position, a highest success route, a least invasive route, or a combination thereof. As shown in the example provided in FIG. 4, the route 410 may be the shortest route from a non-optimal position to the optimal position 312 while avoiding areas 401 and 402 of a heart. The implantable device 320 may be directed along the route 410 (e.g., using a shaft 22 of FIG. 2A or via remote guidance via a network such as network 62) to reach the optimal position 312. If the implantable device 320 deviates from the route 410, the route 410 may be updated based on the deviated position of the implantable device 320.

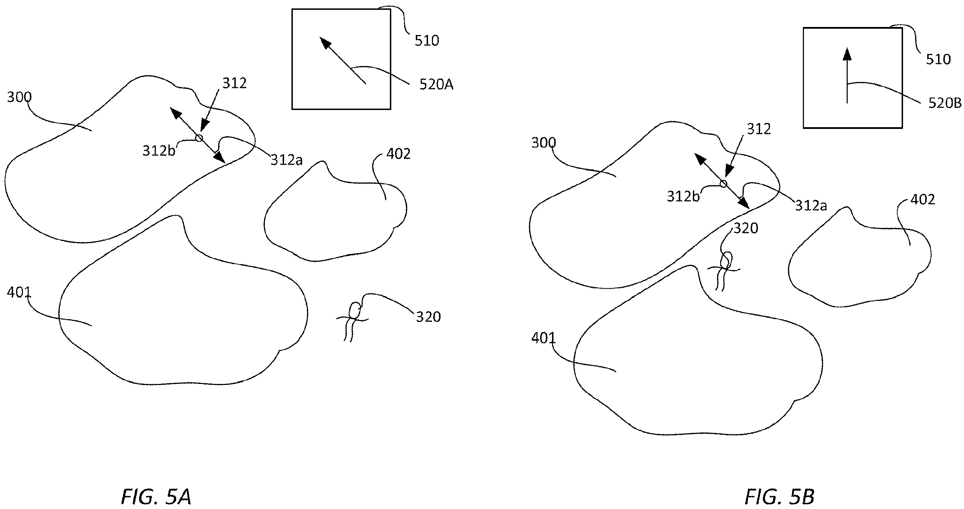

FIGS. 5A and 5B shows the example of heart organ 300 provided in FIG. 4. For conciseness, the components of FIG. 4 that are also provided in FIGS. 5A and 5B are not described again specifically in reference to FIGS. 5A and 5B. As shown in FIG. 5A and 5B, visual directions from a non-optimal position to an optimal position may be provided using a direction panel 510. As shown in FIG. 5A, the direction panel 510 may show a north western direction 520A based on a current non-optimal location of the implantable device 320 in FIG. 5A. The implantable device 320 may move in accordance with the north western direction 520A to reach the non-optimal position as shown in FIG. 5B. Based on the updated position of the implantable device 320, the direction panel 510 may be updated to show the north direction 520B which would enable the implantable device 320 to reach the optimal position 312. The direction panel may also provide an indication of the orientation 312b prior to or after the implantable device 320 reaches the location 312a. Such an orientation indication may be provided, for example, using a different color arrow or any other applicable indication that distinguishes the visual indication for location 312a from the orientation 312b.

It will be understood that although visual directions are provided in FGIS. 5A and 5B, the directions may be other sensory directions such as auditory directions. Further, it will be understood that although an arrow is shown in FIGS. 5A and 5B, the directions may be provided in any other applicable manner such as a compass, a heat map, a three-dimensional rendering, or the like.

According to an implementation, haptic feedback may be provided to guide an implantable device to an optimal position. Such haptic feedback may include a force and/or a resistance that makes it physically easier for an implantable device to be guided towards an optimal position and makes it physically more difficult for the implantable device to be guided in directions that are away from the optimal position.

The haptic feedback may be applied to, for example, a portion of a shaft (e.g., shaft 22 of FIG. 1) that is used to control the direction an implantable device is traversing. The portion of the shaft may provide force feedback that positively reinforces directions that causes the implantable device to reach the optimal position, based on updated current positions of the implantable device. For example, if the optimal position path is to the anatomical right of the current position of the implantable device, the shaft may be configured to provide resistance if a user or automated device directs the implantable device to the anatomical left. Further, the shaft may be configured to provide less or no resistance or may provide an additional force in the anatomic right direction if a user or automated device directs the implantable device to the anatomical right.

The haptic feedback may be applied to, for example, a local or remote guidance tool that may be used to direct the implantable device within a patient's body. The local or remote guidance tool may be, for example, a joystick, a control-pad, an electronic mouse, or the like and may use wired or wireless electronic signals to direct the implantable device. The haptic feedback may be provided using virtual rails that create virtual paths from a non-optimal position to an optimal position via software such that if the implantable device and/or the guiding tool deviate from the virtual path, a force or resistance based haptic feedback directs the implantable device and/or the guiding tool back to the virtual path.

At step 240 of process 200 of FIG. 2A, the implantable device may be implanted at the optimal position. The implantable device may be implanted by an adhesive attachment, tissue insersion, mechanical attachment, or the like. An implanted implantable device may record biometric data at the optimal position and may provide the biometric data to, for example, a local or remote computing device via a wireless signal.

According to implementations of the disclosed subject matter, a remote computing system may be provided. The remote computing system may be utilized to provide a medical condition indication, as described at step 210 of process 200 of FIG. 2A. For example, a physician may remotely diagnose a medical condition based on a patient procedure. The procedure may be performed to treat the medical condition and the medical condition indication may be provided such that an implantable device can be implanted, in accordance with process 200, to monitor the on-going results of the medical procedure.

Further, a remote computing system may be used to guide the implantable device from a non-optimal position to an optimal position, as disclosed at step 230 of the process 200 of FIG. 2A. The remote computing system may include, for example, a remote guidance tool that wirelessly directs an implantable device to an optimal position. A current location of the implantable device may be determined through any applicable technique such as by using one or more coils that may be attached to the implantable device or to a shaft (e.g., shaft 22), by electromagnetic signals, by body surface electrodes, by one or more location pads, or the like.

Further, a remote computing system may be used to receive biometric data from an implanted implantable device and may analyze or otherwise provide the received biometric data to a medical professional.

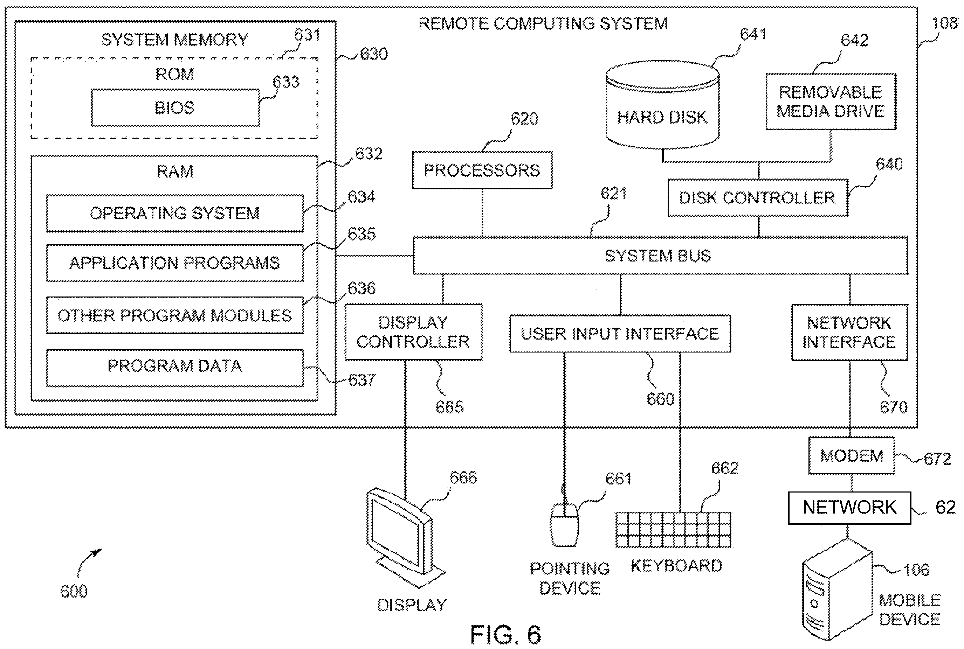

FIG. 6 is a system diagram of an example of a computing environment 600 in communication with network 62 of FIG. 1. In some instances, the computing environment 600 is incorporated in a public cloud computing platform (such as Amazon Web Services or Microsoft Azure), a hybrid cloud computing platform (such as HP Enterprise OneSphere) or a private cloud computing platform.

As shown in FIG. 6, computing environment 600 includes remote computing system 108, which is one example of a computing system upon which embodiments described herein may be implemented.

The remote computing system 108 may, via processors 620, which may include one or more processors, perform various functions. The functions may include analyzing received patient biometrics and the associated information and, according to physician-determined or algorithm driven thresholds and parameters, providing (e.g., via display 666) alerts, additional information or instructions. As described in more detail below, the remote computing system 108 may be used to provide (e.g., via display 666) healthcare personnel (e.g., a physician) with a dashboard of patient information (e.g., information obtained by an implanted implantable device), such that such information may enable healthcare personnel to identify and prioritize patients having more critical needs than others.

As shown in FIG. 6, the remote computing system 108 may include a communication mechanism such as a bus 621 or other communication mechanism for communicating information within the computer system 610. The computer system 610 further includes one or more processors 620 coupled with the bus 621 for processing the information. The processors 620 may include one or more CPUs, GPUs, or any other processor known in the art.

The computer system 610 also includes a system memory 630 coupled to the bus 621 for storing information and instructions to be executed by processors 620. The system memory 630 may include computer readable storage media in the form of volatile and/or nonvolatile memory, such as read only system memory (ROM) 631 and/or random access memory (RAM) 632. The system memory RAM 632 may include other dynamic storage device(s) (e.g., dynamic RAM, static RAM, and synchronous DRAM). The system memory ROM 631 may include other static storage device(s) (e.g., programmable ROM, erasable PROM, and electrically erasable PROM). In addition, the system memory 630 may be used for storing temporary variables or other intermediate information during the execution of instructions by the processors 620. A basic input/output system 633 (BIOS) may contain routines to transfer information between elements within computer system 610, such as during start-up, that may be stored in system memory ROM 631. RAM 632 may contain data and/or program modules that are immediately accessible to and/or presently being operated on by the processors 620. System memory 630 may additionally include, for example, operating system 634, application programs 635, other program modules 636 and program data 637.

The illustrated computer system 610 also includes a disk controller 640 coupled to the bus 621 to control one or more storage devices for storing information and instructions, such as a magnetic hard disk 641 and a removable media drive 642 (e.g., floppy disk drive, compact disc drive, tape drive, and/or solid state drive). The storage devices may be added to the computer system 610 using an appropriate device interface (e.g., a small computer system interface (SCSI), integrated device electronics (IDE), Universal Serial Bus (USB), or FireWire).

The computer system 610 may also include a display controller 665 coupled to the bus 621 to control a monitor or display 666, such as a cathode ray tube (CRT) or liquid crystal display (LCD), for displaying information to a computer user. The illustrated computer system 610 includes a user input interface 660 and one or more input devices, such as a keyboard 662 and a pointing device 661, for interacting with a computer user and providing information to the processor 620. The pointing device 661, for example, may be a mouse, a trackball, or a pointing stick for communicating direction information and command selections to the processor 620 and for controlling cursor movement on the display 666. The display 666 may provide a touch screen interface that may allow input to supplement or replace the communication of direction information and command selections by the pointing device 661 and/or keyboard 662.

The computer system 610 may perform a portion or each of the functions and methods described herein in response to the processors 620 executing one or more sequences of one or more instructions contained in a memory, such as the system memory 630. Such instructions may be read into the system memory 630 from another computer readable medium, such as a hard disk 641 or a removable media drive 642. The hard disk 641 may contain one or more data stores and data files used by embodiments described herein. Data store contents and data files may be encrypted to improve security. The processors 620 may also be employed in a multi-processing arrangement to execute the one or more sequences of instructions contained in system memory 630. In alternative embodiments, hard-wired circuitry may be used in place of or in combination with software instructions. Thus, embodiments are not limited to any specific combination of hardware circuitry and software.

As stated above, the computer system 610 may include at least one computer readable medium or memory for holding instructions programmed according to embodiments described herein and for containing data structures, tables, records, or other data described herein. The term computer readable medium as used herein refers to any non-transitory, tangible medium that participates in providing instructions to the processor 620 for execution. A computer readable medium may take many forms including, but not limited to, non-volatile media, volatile media, and transmission media. Non-limiting examples of non-volatile media include optical disks, solid state drives, magnetic disks, and magneto-optical disks, such as hard disk 641 or removable media drive 642. Non-limiting examples of volatile media include dynamic memory, such as system memory 630. Non-limiting examples of transmission media include coaxial cables, copper wire, and fiber optics, including the wires that make up the bus 621. Transmission media may also take the form of acoustic or light waves, such as those generated during radio wave and infrared data communications.

The computing environment 600 may further include the computer system 610 operating in a networked environment using logical connections to local computing device 106 and one or more other devices, such as a personal computer (laptop or desktop), mobile devices (e.g., patient mobile devices), a server, a router, a network PC, a peer device or other common network node, and typically includes many or all of the elements described above relative to computer system 610. When used in a networking environment, computer system 610 may include modem 672 for establishing communications over a network 120, such as the Internet. Modem 672 may be connected to system bus 621 via network interface 670, or via another appropriate mechanism.

Network 62, as shown in FIGs. 1 and 6, may be any network or system generally known in the art, including the Internet, an intranet, a local area network (LAN), a wide area network (WAN), a metropolitan area network (MAN), a direct connection or series of connections, a cellular telephone network, or any other network or medium capable of facilitating communication between computer system 610 and other computers (e.g., local computing device 106).

Any of the functions and methods described herein can be implemented in a general-purpose computer, a processor, or a processor core. Suitable processors include, by way of example, a general purpose processor, a special purpose processor, a conventional processor, a digital signal processor (DSP), a plurality of microprocessors, one or more microprocessors in association with a DSP core, a controller, a microcontroller, Application Specific Integrated Circuits (ASICs), Field Programmable Gate Arrays (FPGAs) circuits, any other type of integrated circuit (IC), and/or a state machine. Such processors can be manufactured by configuring a manufacturing process using the results of processed hardware description language (HDL) instructions and other intermediary data including netlists (such instructions capable of being stored on a computer-readable media). The results of such processing can be maskworks that are then used in a semiconductor manufacturing process to manufacture a processor which implements features of the disclosure.

Any of the functions and methods described herein can be implemented in a computer program, software, or firmware incorporated in a non-transitory computer-readable storage medium for execution by a general-purpose computer or a processor. Examples of non-transitory computer-readable storage mediums include a read only memory (ROM), a random access memory (RAM), a register, cache memory, semiconductor memory devices, magnetic media such as internal hard disks and removable disks, magneto-optical media, and optical media such as CD-ROM disks, and digital versatile disks (DVDs).

It should be understood that many variations are possible based on the disclosure herein. Although features and elements are described above in particular combinations, each feature or element can be used alone without the other features and elements or in various combinations with or without other features and elements.

Aspects of the invention

1. A method comprising:

receiving a medical condition indication;
determining an optimal position for an implantable device based on the medical condition indication;
guiding the implantable device from a non-optimal position to the optimal position based on determining the optimal position; and
implanting the implantable device at the optimal position.

2. The method of aspect 1, wherein receiving the medical condition indication comprises receiving the medical condition indication from a remote computing system.

3. The method of aspect 1, wherein the medical condition indication may be at least one of a numerical value, an electronic signal, and a code value.

4. The method of aspect 1, wherein the medical condition indication is one of automatically determined and user provided.

5. The method of aspect 1, wherein the optimal position comprises at least one of a location and an orientation.

6. The method of aspect 1, wherein the optimal position is based on an identified problem location.

7. The method of aspect 6, wherein the identified problem location is determined based on at least one of an estimated arrhythmia and an identified arrhythmia.

8. The method of aspect 1, wherein determining the optimal position for the implantable device further comprises determining one or more of potential signal to noise ratios, potential signal amplitudes, and potential morphologies.

9. The method of aspect 1, wherein guiding the implantable device comprises providing a visual path from the non-optimal position to the optimal position.

10. The method of aspect 1, wherein guiding the implantable device comprises providing a direction towards the optimal position.

11. The method of aspect 1, wherein guiding the implantable device comprises force directing the implantable device towards the optimal position.

**Claims**

1. A system comprising:

an implantable device configured to implant inside a human body;
a processor configured to:

receive a medical condition indication,
determine an optimal position for the implantable device based on the medical condition indication,
receive a non-optimal position of the implantable device, and
guide the implantable device from the non-optimal position to the optimal position; and

a remote computing system configured to receive data from the implantable device at the optimal position.

2. The system of claim 1, wherein receiving the medical condition indication comprises receiving the medical condition indication from at least one of a diagnosis system, a processor, and a user.

3. The system of claim 1, wherein the medical condition indication may be at least one of a numerical value, an electronic signal, and a code value.

**4.** The system of claim 1, wherein the medical condition indication is one of automatically determined and user provided.

**5.** The system of claim 1, wherein the optimal position comprises at least one of a location and an orientation.

**6.** The system of claim 1, wherein the optimal position is based on an identified problem location.

**7.** The system of claim 1, wherein determining the optimal position for the implantable device further comprises determining one or more of potential signal to noise ratios, potential signal amplitudes, and potential morphologies.

**8.** The system of claim 1, wherein guiding the implantable device comprises at least one of providing a visual path from the non-optimal position to the optimal position, providing a direction towards the optimal position and force directing the implantable device towards the optimal position.

**9.** The system of claim 1, wherein the remote computing system is further configured to update the position of the implantable device based on the received data.

FIG. 1

200

EP 3 675 136 A1

```
┌─────────────────────────────────────┐
│  Receive a medical condition         │ ── 210
│  indication                          │
└─────────────────────────────────────┘
                    │
                    ▼
┌─────────────────────────────────────┐
│  Determine an optimal position for an│
│  implantable device based on the     │ ── 220
│  medical condition indication        │
└─────────────────────────────────────┘
                    │
                    ▼
┌─────────────────────────────────────┐
│  Guide the implantable device from a │
│  non-optimal position to the optimal │ ── 230
│  position based on determining the   │
│  optimal position                    │
└─────────────────────────────────────┘
                    │
                    ▼
┌─────────────────────────────────────┐
│  Implant the implantable device at   │
│  the optimal position                │ ── 240
└─────────────────────────────────────┘
```

*FIG. 2A*

```
┌─────────────────────────────────────┐
│  Apply weights to a plurality of factors │──── 252
└─────────────────────────────────────┘
                    │
                    ▼
┌─────────────────────────────────────┐
│     Assign score to multiple anatomical  │
│  positions based on the weights applied to │──── 254
│         the plurality of factors          │
└─────────────────────────────────────┘
                    │
                    ▼
┌─────────────────────────────────────┐
│   Determine optimal position based on the │
│    anatomical position with the highest   │──── 256
│                 score                     │
└─────────────────────────────────────┘
```

*FIG. 2B*

FIG. 3

EP 3 675 136 A1

FIG. 4

*FIG. 5B*

*FIG. 5A*

FIG. 6

Europäisches
Patentamt

European
Patent Office

Office européen
des brevets

# EUROPEAN SEARCH REPORT

Application Number

EP 19 22 0106

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2018/220970 A1 (KAHWASH RAMI [US]) 9 August 2018 (2018-08-09) * paragraph [0002] - paragraph [0014] * * paragraph [0022] - paragraph [0032] * ----- | 1-9 | INV. G16H40/63 G16H50/20 G16H50/70 G16H40/67 |
| Y | WO 2018/200767 A1 (ARTHROLOGY CONSULTING LLC [US]) 1 November 2018 (2018-11-01) * abstract; figure 8 * * paragraph [0061] - paragraph [0065] * ----- | 1-9 | G16H20/40 G16H50/50 |

TECHNICAL FIELDS
SEARCHED (IPC)

G16H

The present search report has been drawn up for all claims

| Place of search | Date of completion of the search | Examiner |
|---|---|---|
| Munich | 12 May 2020 | Menschner, Philipp |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 22 0106

This annex lists the patent family members relating to the patent documents cited in the above-mentioned European search report.
The members are as contained in the European Patent Office EDP file on
The European Patent Office is in no way liable for these particulars which are merely given for the purpose of information.

12-05-2020

| Patent document cited in search report | | Publication date | Patent family member(s) | | Publication date |
|---|---|---|---|---|---|
| US 2018220970 | A1 | 09-08-2018 | EP | 3307212 A1 | 18-04-2018 |
| | | | US | 2018220970 A1 | 09-08-2018 |
| | | | WO | 2016201336 A1 | 15-12-2016 |
| WO 2018200767 | A1 | 01-11-2018 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62786836